# EUROPEAN PATENT SPECIFICATION

(11) **EP 0 631 472 B1**
(45) Date of publication and mention of the grant of the patent: **12.08.1998**
(21) Application number: 93908378.8
(22) Date of filing: 18.03.1993
(51) Int. Cl.: A01N 25/08, A01N 47/44, A61F 6/04

(54) **ANTIVIRAL CONDOMS**
ANTIVIRALE KONDOME
DISPOSITIFS ANTIVIRAUX

(30) Priority: 18.03.1992 US 853535
(43) Date of publication of application: 04.01.1995
(73) Proprietor: The Trustees of Columbia University in the City of New York, New York, NY 10026-6699 (US)
(72) Inventor: MODAK, Shanta, M., River Edge, NJ 07661 (US); SAMPATH, Lester, Nyack, NY 10960 (US)
(74) Representative: Schwarz, Albin, Dr. Kopecky & Schwarz Patentanwälte
(86) International application number: US9302436
(87) International publication number: WO9318650

(56) References cited:
- EP-A- 0 247 251
- WO-A-87/00005
- WO-A-90/04390
- WO-A-93/15728
- US-A- 4 772 470
- US-A- 4 836 853
- US-A- 5 013 544
- US-A- 5 073 365
- US-A- 5 089 205
- J. Antibact. Antifung Agents, Vol. 16, No. 11, (1988), (YAMAMOTO), "Benzalkonium Chloride, Chlorhexidine Digluconate, Human Immunodeficiency Virus", pages 505-508.
- Journal of Acquired Immune Deficiency Syndromes, 2:16-20 (1989) (HARBISON), "Inactivation of Human Immunodeficiency Virus by Betadine - Products and Chlorhexidine", see entire document.

## Description

### Background of the Invention

This application relates to antiinfective condoms having a quick release coating containing a biguanide antiinfective agent. The condoms of the invention provide rapid protection against infectious agents, including viruses, and are stable over prolonged storage periods.

Condoms provide a barrier function to prevent passage of sperm. At the same time, a barrier to the passage of infectious microorganisms may be provided in a manner similar to the way in which surgical and examination gloves provide a barrier function providing separation between a patient and a health care worker. In fulfilling this function, the condoms act to block the introduction of infectious agents, particularly viruses, bacteria and fungi from the body fluids of one individual to another.

Of particular significance in this regard are viruses, such as HIV, the virus causing Acquired Immunodeficiency Syndrome (AIDS), and Hepatitis B virus (HBV) which may even penetrate through a latex condom that is not actually perforated but merely stretched, although other organisms causing sexually transmitted diseases (STD's) are also of significance. Agents which are effective against these pathogenic agents, however, are less common than those that will provide an effect against simple skin bacteria or fungi and must frequently be present at much higher levels to be efficacious. This can cause difficulties if the user is in contact with high levels of antiinfective agent for prolonged periods of time. It would therefore be highly advantageous to provide condoms in which an effective virucidal agent were maintained in a "ready" state, available for quick or even instant release as needed to counter the effects of possible viral contamination.

Chlorhexidine has the ability to kill the AIDS virus and HBV, as well as numerous other STD causing organisms, as shown in prior commonly assigned U.S. Patent No. 5,019,096 and 4,952,411 and application Serial No. 07/385,290, which are incorporated herein by reference. The utility of nonoxynol-9 as an antiseptic, spermicide and antiviral agent is described in U.S. Patent No. 5,073,365. EP-A - 0 247 251 and U.S. Patent No. 5,013,544 describe synergistic spermicidal or sperm-immobilizing effects of mixtures of nonoxynol-9 and chlorhexidine. Previous disclosures of the use or chlorhexidine in medical gloves, e.g. in U.S. Patent No. 4,853,978, and the use of chlorhexidine gluconate and nonoxynol-9 in vaginal contraceptives, particularly diaphragms, in EP-A - 0 247 251 provide release from the coating over several hours, and thus are not quick enough to provide meaningful protection from viral pathogens. Moreover, we have found that gloves made by dipping cured gloves in an antimicrobial preparation suffer from significant activity loss on storage, and thus from poor reliability. This deficiency would extend to condoms given the similarity of the base materials. WO 90/04390 describes an antiviral composition which can be coated on a condom comprising a biguanide, such as chlorhexidine or a salt thereof, a silver salt and optionally additional antiinfective materials, such as nonoxynol. U.S. Patent No. 5,089,205 describes a method for producing medical devices, among them condoms, having antimicrobial properties by coating the surface of the medical device with a composition of an antimicrobial surfactant and a chlorhexidine base or its salts. The medical device can be made of latex, or a non-latex elastomer, which is treated with a polyurethane dispersion or solution prior to its being coated with the composition.

It is the objective of the present invention to provide condoms which rapidly release effective antiviral amounts of an antiinfective agent upon exposure to liquid, and which retain this ability over periods of prolonged storage.

### Summary of the Invention

In accordance with the present invention, an antiviral elastomeric condom is obtained by preventing adsorption of the antiinfective agent by the lubricating agent and optionally by the elastomeric material itself. The condom of the invention has interior and exterior surfaces and an antiinfective coating disposed on the interior surface, wherein the antiinfective coating comprises (a) an antiinfective agent selected from the group consisting of chlorhexidine and pharmaceutically acceptable salts of chlorhexidine and (b) nonoxynol-9, wherein the antiinfective agent and nonoxynol-9 are present in a ratio between 8:1 and 4:1 by weight, respectively, said antiinfective coating being effective to deliver an antivirally effective amount of the antiinfective agent within ten minutes of exposure to a physiologic liquid.

### Detailed Description of the Invention

The present invention resides in an antiviral condom. The condom of the invention comprises a shaped, elastomeric body and an antiinfective coating.

The elastomeric body may be formed from any of a variety of materials known in the art for the manufacture of elastomeric articles such as condoms including polyvinylchloride, polyurethane and silicone rubbers. Natural rubber latex is the preferred material, however, because of the flexibility and durability of this material.

The elastomeric body is formed in accordance with known procedures for the formation of condoms. Basically, these procedures involve preparing a fluid containing the elastomer, dipping a shaped mandrel into the fluid to obtain a shaped coating, and coagulating, drying and curing the coating. The desired inner coating can be incorporated on the outside of the coated mandrel, either before or after the curing step, because the condom is inverted in the process of removing it from the mandrel.

The antiinfective coating of the invention comprises chlorhexidine or a pharmaceutically acceptable salt of chlorhexidine as an antiinfective agent. Suitable salts of chlorhexidine include chlorhexidine gluconate, chlorhexidine acetate and chlorhexidine chloride.

The antiinfective coating of the invention also includes nonoxynol-9 (e.g. 0,1 to 0,5 mg per condom) as a lubricating agent. This lubricating agent is selected so that it does not significantly adsorb the antiinfective agent as this adsorption retards the release of the antiinfective agent, e.g., as in the Stockum glove patent, U.S. 4,583,978.

The antiinfective coating may also incorporate one or more biomedically acceptable polymers. Suitable materials include polyurethanes and silicones which are dealt with at length in U.S. Patent Application S.N. 07/385,290 cited hereinabove. The use of these materials may be desirable to minimize the possibility of lubricating agent being released from the condom surface and to provide lubricity due to the nature of the polymeric component. Additional polymer materials may also reduce binding of the antiinfective agent to the latex, as these materials have less affinity for the antiinfective agent such that even if bound, the antiinfective agent is rapidly released from this surface.

The antiinfective coating is formulated using nonoxynol-9 and sufficient antiinfective agent such that an effective antiviral amount of the antiinfective agent is released within ten minutes of being exposed to a fluid, e.g., semen or other body fluid. Preferably the antiinfective coating will provide substantially instantaneous release of the antiinfective agent so that any virus or other infective agent present is killed in the minimum possible time. We have found that concentrations of 300 µg/ml of chlorhexidine are sufficient to prevent infectivity by HBV or Rauscher's Leukemia Virus, an accepted model for HIV. Thus, suitable antiviral levels would be achieved if there were about 1.25 to 2.5 mg of releasable antiinfective agent per condom. To provide protection to the female in case of leakage of ejaculate from the condom, however, it is desirable to include an additional amount of the antiinfective lubricant composition near the tip of the condom, for example an additional amount containing from 2.0 to 6.0 mg of chlorhexidine. Thus, the total amount of releasable chlorhexidine is preferably from 1.25 to 8.5 mg per condom, and more preferably from 3.25 to 8.5 mg.

In addition to adsorption by the lubricating agent, the antiviral agent may also be adsorbed or otherwise made unavailable for release by the shaped, elastomeric body. This is particularly significant in the case of natural rubber articles, because natural rubber latex has a high affinity for chlorhexidine. This type of adsorption appears to be a major fact or in loss of activity on storage of chlorhexidine treated gloves. Specifically, it appears that chlorhexidine originally present in the inner coating may be taken up over time by the glove body to be released slowly, if at all, on contact with fluids.

This problem of adsorption of the antiviral agent by the body of the article leading to poor shelf stability can be solved in two ways. The first involves the manufacturing procedure of the article, the second incorporation of an additional material in the article.

In the first approach to producing condoms with high shelf stability, the devices are cast onto the mandrels and dried to form a film in the normal manner. Then, however, prior to curing the elastomeric body, the body is soaked in a solution of antiviral agent to saturate its ability to adsorb the antiviral agent. This saturation process may be accelerated by the heating, e.g., to 100°C. The antiviral agent in the subsequently applied antiinfective coating cannot be adsorbed by the saturated elastomeric body and remains available at a consistent level throughout the shelf life of the condom, generally a period of 6 months or longer.

While this approach to saturating the elastomeric body is effective it has two potential drawbacks. First, a high level of antiinfective agent is actually present in the condom which may be released if the device is worn for long periods of time. Secondly, using the antiinfective agent itself as the saturating agent is not very cost effective. For these reasons, it may be desirable to saturate adsorption sites in the condom body with a distinct material for this purpose. Suitable materials include metal ions or heavy metals such as zinc, silver, etc.; organic acids such as gluconic acid and surfactants preferably cationic surfactants, such as quaternary ammonium compounds.

Adsorption of antiviral agent by the elastomeric body can also be prevented by covering the surface of the body with a thin layer of a lubricating agent/surfactant - treated silicone or polyurethane, and then coating with the antiinfective coating player containing the antiinfective agent and nonoxynol-9. Preferred materials for this thin layer are 2-5% silicone emulsion treated with didecyldimethylammonium chloride (sold under the trade name Bardac) or a 10% polyurethane. Adsorption by the elastomeric body can also be prevented by the use of weak acid and Bardac before high temperature cure. A suitable treatment involves application of 0.3% gluconic acid and 0.2% Bardac before the antiinfective agent.

In the case where a separate saturating material is used, this agent is advantageously added either to the original fluid for molding into the elastomeric or between the drying and curing steps. The coating containing the antiviral itself is then applied either before or after curing of the glove body.

If polymeric materials such as silicones are to be included in the antiinfective coating, the elastomeric body should be saturated to block adsorption and the antiinfective coating should be applied after curing of the elastomeric body. In this way, reaction of the antiviral agent with the silicone polymer which might occur at the curing temperatures, thus bonding the antiviral in the inner coating and eliminating the quick release performance features of the contraceptive device is eliminated.

The invention will now be further described by way of the following specific examples. These examples are intended to demonstrate the efficacy of the invention and are not intended to limit the scope of protection.

### Example 1

### Method of Preparation of Condoms with Chlorhexidine

Condom forms are dipped in a coagulant bath (e.g., 29 gm of a mixture of calcium carbonate and calcium nitrate and 0.1 ml of Surfynol TG surfactant in 400 ml deionized water) at 50°C for 24 seconds. The forms are then dried for about 75 seconds at 100°C to prepare them for dipping in the latex dipping solution.

A 30% latex emulsion is prepared by mixing 200 ml of 60% TSC concentrate latex with 200 ml deionized water containing 0.16 ml of Bevaloid and stirred gently on a magnetic stirrer. The pH was adjusted to 10.0 using ammonium hydroxide. The coagulant-coated formers are then dipped in latex solution for 15 seconds after which the formed condoms are dried at 100°C for 5 minutes.

The condoms are then immersed in a water bath at 80°C for 3 minutes to leach out unwanted chemicals. In an industrial setting, the standard procedure for preparing the condoms is carried out up to the leaching step.

After leaching, the condoms are cured at 100°C for one-half hour. As soon as the condoms come out of the oven and while they are hot, they are dipped into a slurry containing 3% chlorhexidine + 5% zinc oxide + 1% glycerine + 0.7 gm aqueous based lubricant, e.g. nonoxynol-9 containing 1,000 µg CHG.

### Example 2

Condoms prepared by dipping the leached condoms of Example 1 into a solution containing 0.5% to 5.0% chlorhexidine gluconate and 5.0% to 10.0% cornstarch containing 0.02% to 0.2% Bardac and curing at 100°c for one-half hour were used for evaluation of bioactivity.

The effect of CHG coated condoms and nonoxynol-9 coated condoms prepared as above + 0.7 % nonoxynol-9 as to lubrication inner surface) were evaluated for their antifungal activity in the presence of trypticase soy broth containing 12.5% albumin. The results are given in Table 1.

**TABLE 1**

| Antifungal Activity of CHG-Coated and Nonoxynol-9 Coated Condoms | | |
|---|---|---|
| Group | Colony Forming Units | |
| | 5 Minutes Exposure | 10 Minutes Exposure |
| Control Condom | 1.0 × 10⁵ | 1.0 × 10⁵ |
| Condom with 10 mg Nonoxynol-9 | 4.0 × 10⁵ | 2.2 × 10⁵ |
| Condom with 20 mg Nonoxynol-9 | 1.2 × 10⁵ | 1.2 ×10⁵ |
| Condom with 500 µg CHG | 3.6 × 10² | 1.8 × 10² |
| Condom with 1500 µg | 0 | 0 |

### Example 3

### Effect of CHG-Coated Condoms on the Lymphocytes and Macrophages in Semen

It is well documented that HIV in semen is mainly associated with lymphocytes and macrophages, with relatively few viruses detected in semen plasma. In order to inactivate the virus, the cells with which they are associated have to be disrupted first to get access to the virus. We have shown that the lethal concentration of chlorhexidine for lymphocytes in blood is 3000 µg for 10⁴ cells/ml and 300 µg/ ml for HIV.

The disintegration of lymphocytes and macrophage can result in the complete destruction of viruses, therefore, the effect of chlorhexidine on these cells in the presence of semen was evaluated to determine whether a chlorhexidine coated condom could inactivate the cell associated viruses in the ejaculate.

Lymphocytes from blood were separated on a Ficoll Hypaque gradient. To different 1 ml samples of semen 10⁴ lymphocytes were added and exposed to different amounts of chlorhexidine and nonoxynol-9 and immediately diluted 30-fold with phosphate buffered saline (PBS). 3 ml was then layered on 3 ml Ficoll Hypaque gradient and centrifuged at 400 × g for 30 minutes. After centrifugation, the upper layer to within 0.5 cm of the opaque interface containing lymphocytes was aspirated with a Pasteur pipette and discarded. The opaque layer of lymphocytes on the corresponding region on the gradient was carefully transferred to a conical centrifuge tube. 5 ml PBS was added to this tube, mixed well and centrifuged at 250 × g for 10 minutes. The supernatant was discarded and the lymphocyte pellet was obtained after centrifugation, resuspended in PBS, centrifuged and the pellet collected. Lymphocyte vialibity was determined by exclusion of trypan blue. The cidal effect of chlorhexidine, nonoxynol-9 and the combination of the two on the lymphocytes in semen was determined using the above procedure and the results are shown in Table 2.

The results in Table 2 show that 2000 µg chlorhexidine gluconate inactivates 97% of the lymphocytes when exposed to 1 ml semen containing 10⁴ cells. The addition of small amounts of nonoxynol-9 to chlorhexidine appears to exhibit synergism.

**TABLE 2**

| Inactivation of Lymphocytes in Semen By Chlorhexidine and Nonoxynol-9 | | | |
|---|---|---|---|
| Drug (µg/ml) | % Viable Cells | % Non-Viable Cells | % Disintegrated Cells |
| CHG - 4000 | 0 | 0 | 100 |
| CHG - 2000 | 3 | 5 | 92 |
| CHG - 1000 | 33 | 18 | 49 |
| NN-9 250 | 28 | 18 | 54 |
| NN-9 2000 | 0 | 0 | 100 |

| CHG-NN-9 | | | |
|---|---|---|---|
| 2000 + 250 | 0 | 0 | 100 |
| 1000 + 250 | 2 | 10 | 88 |
| Control (No Drug) | 63 | 37 | 0 |
| Lymphocyte counts in semen 5 × 10⁴/ml. | | | |

## Claims

1. A condom having interior and exterior surfaces and an antiinfective coating disposed on the interior surface, wherein the antiinfective coating comprises (a) an antiinfective agent selected from the group consisting of chlorhexidine and pharmaceutically acceptable salts of chlorhexidine and (b) nonoxynol-9, wherein the antiinfective agent and nonoxynol-9 are present in a ratio between 8:1 and 4:1 by weight, respectively, said antiinfective coating being effective to deliver an antivirally effective amount of the antiinfective agent within ten minutes of exposure to a physiologic liquid.

2. The condom of claim 1 in which the antiinfective agent is chlorhexidine and the amount of releasable chlorhexidine is between about 1.25 milligrams and 8.5 milligrams.

3. The condom of claim 1 which is formed from natural rubber latex and in which the natural rubber latex is modified to saturate binding sites for the antiinfective agent in the latex.

4. The condom of claim 2 which is formed from natural rubber latex and in which the natural rubber latex is modified to saturate binding sites for the antiinfective agent in the latex.

## Patentansprüche

1. Kondom mit innerer und äußerer Oberfläche und einer auf der inneren Oberfläche angeordneten antiinfektiven Beschichtung, wobei die antiinfektive Beschichtung (a) ein antiinfektives Mittel, ausgewählt aus der Gruppe bestehend aus Chlorhexidin und pharmazeutisch annehmbaren Salzen von Chlorhexidin, und (b) Nonoxynol-9 umfaßt, wobei das antiinfektive Mittel und Nonoxynol-9 in einem Gewichtsverhältnis zwischen 8:1 und 4:1 vorliegen und die antiinfektive Beschichtung so wirksam ist, daß sie innerhalb einer zehnminütigen Einwirkung einer physiologischen Flüssigkeit eine antiviral wirksame Menge des antiinfektiven Mittels freisetzt.

2. Kondom gemäß Anspruch 1, bei dem das antiinfektive Mittel Chlorhexidin ist und die Menge an freisetzbarem Chlorhexidin zwischen etwa 1,25 Milligramm und 8,5 Milligramm beträgt.

3. Kondom gemäß Anspruch 1, welches aus Naturlatex gebildet ist und bei dem der Naturlatex modifiziert ist, um Bindungsstellen für das antiinfektive Mittel im Latex abzusättigen.

4. Kondom gemäß Anspruch 2, welches aus Naturlatex gebildet ist und bei dem der Naturlatex modifiziert ist, um Bindungsstellen für das antiinfektive Mittel im Latex abzusättigen.

## Revendications

1. Préservatif ayant des surfaces intérieure et extérieure et une couverture anti-infectieuse déposée sur la surface intérieure, dans lequel la couverture anti-infectieuse comporte (a) un agent anti-infectieux choisi parmi le groupe consistant en chlorhexidine et les sels pharmaceutiquement acceptables de la chlorhexidine, et (b) du nonoxynol-9, cependant que l'agent anti-infectieux et le nonoxynol-9 sont présents dans un rapport entre 8:1 à 4:1 en poids, respectivement, la couverture anti-infectieuse étant efficace pour délivrer une quantité antiviralement efficace de l'agent anti-infectieux dans dix minutes d'exposition à un liquide physiologique.

2. Préservatif selon la revendication 1, dans lequel l'agent anti-infectieux est la chlorhexidine et la quantité de chlorhexidine capable d'être libérée est de 1,25 mg à 8,5 mg environ.

3. Préservatif selon la revendication 1 qui est formé de latex de caoutchouc naturel et dans lequel le latex de caoutchouc naturel est modifié en vue de saturer des sites de liaison pour l'agent anti-infectieux dans le latex.

4. Préservatif selon la revendication 2 qui est formé de latex de caoutchouc naturel et dans lequel le latex de caoutchouc naturel est modifié en vue de saturer des sites de liaison pour l'agent anti-infectieux dans le latex.
